# DEMANDE DE BREVET EUROPEEN

(11) **EP 0 568 491 A1**
(43) Date de publication de la demande: **03.11.1993**
(21) Numéro de dépôt: 93810053.4
(22) Date de dépôt: 27.01.1993
(51) Int. Cl.: A61M 3/02

(54) **Appareil pour le rinçage des plaies en chirurgie**

(30) Priorité: 01.05.1992 CH 1402/92
(71) Demandeur: Klaiber, Christian, CH-3270 Aarberg (CH)
(72) Inventeur: de Salis, Sker Jean-Rodolphe, CH-2006 Neuchatel (CH); Klaiber, Christian, CH-3270 Aarberg (CH)
(74) Mandataire: North, Mathieu

(57) **Abrégé**

Ce dispositif de nettoyage des plaies pendant les opérations chirurgicales est constitué d'une double chambre étanche à parois verticales souples. La première chambre étanche (12) est séparée de la seconde (13) par deux parois intermédiaires (14). La première chambre (12) reçoit le liquide nettoyant (17). La seconde chambre étanche (13) présente un orifice (4) destiné à être branché sur un circuit d'air comprimé. Une fermeture rapide (6) permet de régler le débit d'air comprimé et de l'arrêter. L'introduction d'air comprimé dans la seconde chambre étanche gonfle cette dernière et exerce une pression sur la seconde, et par conséquent sur le liquide nettoyant. Le liquide est dirigé par un tuyau (5) dans une buse maniée par le chirurgien qui peut nettoyer la plaie au moyen du jet ainsi obtenu. Un tuyau semi-souple (15) est placé entre les parois intermédiaires (14), et se termine par un sifflet (18). Si de l'air comprimé s'échappe de la seconde chambre étanche dans l'interstice entre les parois intermédiraires, l'air passe dans le sifflet et donne l'alerte. Le risque d'avoir des bulles dans le liquide nettoyant est ainsi évité.

## Description

Au cours d'interventions chirurgicales, il faut fréquemment procéder à des nettoyages par rinçage de la zone opératoire au moyen d'un liquide aseptique pour en assurer la désinfection et la propreté.

Les appareils de rinçage et d'aspiration actuels exigent une manutention délicate et leur emploi ne satisfait pas pleinement aux exigences de l'efficacité et de I'ergonomie. Le nettoyage par rinçage est réalisé au moyen d'une buse, ou gicleur, qui termine un tuyau souple relié à un réservoir contenant le liquide aseptique. Le réservoir a la forme d'un sac souple étanche, généralement transparent, en matière synthétique. Le sac est suspendu au-dessus du patient, de sorte que la pression nécessaire est assurée par gravité, le tuyau souple étant relié au bas du sac étanche et le patient étant placé à un niveau inférieur à celui du réservoir. Ce dispositif présente le défaut que la pression est déterminée, et par conséquent limitée, par la hauteur à laquelle il est possible de placer le réservoir; de plus, cette pression diminue à mesure que le liquide s'écoule, de sorte que le chirurgien en train d'opérer ne peut pas compter sur un jet continu de force constante. Il n'a pas la possibilité d'augmenter la pression, si nécessaire.

Il existe bien des solutions consistant à placer le sac souple dans un manchon gonflable, ce qui permet de pallier l'inconvénient cité, mais la manutention de tels dispositifs est peu pratique. Il est en effet nécessaire d'introduire le sac à l'intérieur du manchon et de l'en extraire une fois utilisé le liquide contenu dans le réservoir. Le remplacement du sac utilisé par un sac plein exige en outre que l'on dégonfle préalablement le manchon gonflable. De telles manipulations prennent un temps qui peut être précieux. En outre, en raison de la forme du manchon, le sac souple n'est jamais complètement vide lorsque le manchon est arrivé à son point de gonflement maximal.

Une autre solution a été proposée dans le brevet US 3 949 753 Dockhorn, qui décrit un réservoir à parois élastiquement déformables contenant un liquide aseptique de nettoyage, ce réservoir étant lui-même contenu dans un conteneur dans lequel est injecté de l'air ou de l'eau sous pression. Sous la pression, les parois du réservoir s'enfoncent et le liquide aseptique est éjecté dans un tuyau conduisant à la buse de rinçage. L'inconvénient d'un tel système réside également dans la nécessité d'introduire le réservoir à parois souples dans le conteneur et de l'en extraire une fois qu'il est vide.

Ces deux solutions présentent en outre l'inconvénient d'une fabrication relativement complexe.

La présente invention vise à fournir un dispositif simple et facile à manipuler, qui donne une pression du jet suffisamment élevée et réglable; l'invention vise en outre à fournir un dispositif de faible coût.

L'invention a trait à un dispositif de nettoyage des plaies en chirurgie, comprenant au moins une première chambre étanche apte à contenir un liquide aseptique de nettoyage, au moins une seconde chambre étanche pourvue d'au moins un orifice permettant l'introduction d'un fluide sous pression, au moins un tuyau destiné à conduire le liquide à au moins une buse de giclage, la première et la seconde chambre étanches étant séparées l'une de l'autre par au moins une paroi souple, le dispositif comprenant en outre des moyens d'introduire un fluide sous pression dans la seconde chambre et d'exercer ainsi une pression sur la première chambre par l'intermédiaire de la ou des parois souples et par conséquent sur le liquide aseptique de nettoyage contenu dans la première chambre, les parois des deux chambre étanches étant entièrement souples.

Dans une première forme d'exécution de l'invention, les deux chambres étanches et la ou les parois qui les séparent sont constituées de feuilles étanches souples et réunies par leur pourtour.

Dans une deuxième forme d'exécution, les parois des chambres étanches sont planes lorsque les chambres sont vides.

Dans une troisième forme d'exécution, qui peut se combiner avec les deux formes particulières ci-dessus, ou avec tout dispositif correspondant à la définition générale donnée plus haut, l'orifice permettant l'introduction d'un fluide sous pression dans la seconde chambre est conformé de façon à pouvoir être branché sur un circuit d'air comprimé.

Dans une quatrième forme d'exécution applicable à toutes celles qui sont décrites plus haut, ainsi qu'à tout dispositif correspondant à la définition générale donnée plus haut, la première et la seconde chambres étanches sont séparées par au moins deux parois souples et le dispositif comprend en outre un élément d'alarme apte à fonctionner en cas d'intrusion de fluide entre lesdites parois souples de séparation.

Dans une cinquième forme d'exécution applicable de la même manière, l'élément d'alarme est acoustique.

Dans une sixième forme d'exécution, applicable à toutes les formes ci-dessus mentionnées, l'élément d'alarme est un sifflet.

Dans une septième forme d'exécution, également applicable à toutes celles qui sont mentionnées ci-dessus, et qui constitue la forme d'exécution préférée, au moins l'une des faces des parois souples de séparation qui sont en vis-à-vis a une surface granuleuse.

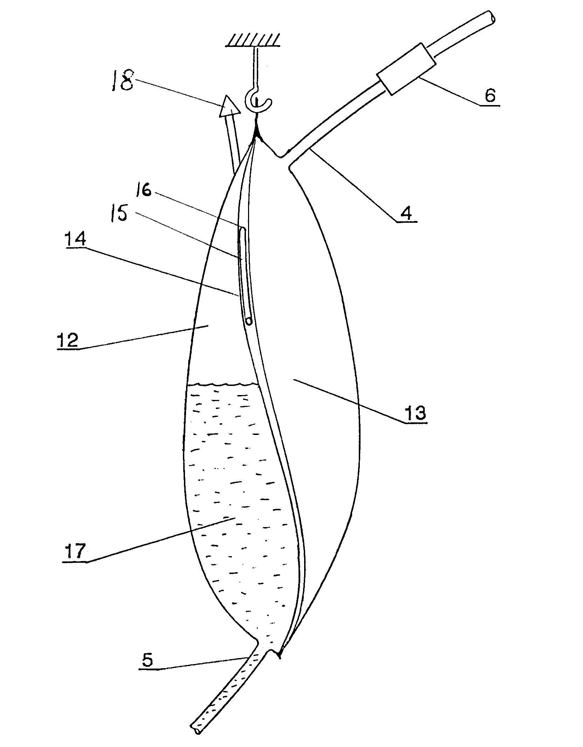

Le dessin montre, à titre d'exemple, une forme d'exécution de l'invention.

La figure représente une vue en coupe verticale de l'appareil selon l'invention.

Le dispositif comprend, comme dans les dispositifs classiques, au moins une première chambre étanche 12. Cette chambre est entièrement souple. Elle est en outre transparente. Dans la forme d'exécution représentée dans le dessin, il n'y a qu'un sac étanche contenant le liquide aseptique. Il est toutefois également concevable d'utiliser un dispositif présentant plusieurs chambres étanches contenant, par exemple, chacune un liquide différent. Le liquide aseptique peut s'écouler par un tube 5 situé au bas de la chambre étanche. Il pourrait y avoir plusieurs tubes de ce genre.

A côté de la première chambre étanche 12 est placée une seconde chambre étanche 13, dont les parois sont également souples. Cette seconde chambre est également transparente dans la forme d'exécution représentée ici.

La seconde chambre peut être fabriquée indépendamment de la première et ensuite plaquée, par exemple par collage du pourtour, contre le premier sac étanche. Dans la forme d'exécution représentée ici, la première et la seconde chambre sont constituées, dans leur ensemble, de quatre parois, c'est-à-dire de deux parois externes et de deux parois intermédiaires 14, les parois intermédiaires séparant le premier sac étanche contenant le liquide du second sac étanche. Comme les deux parois extérieures, les parois intermédiaires sont souples.

La seconde chambre présente au moins un orifice 4, pourvu d'une fermeture rapide 6. La seconde chambre étanche 13 est destinée à recevoir un fluide sous pression. Ce fluide sous pression peut être un liquide, par exemple de l'eau, mais il s'agit de préférence d'un gaz, par exemple de l'air comprimé. Le fluide sous pression est introduit par l'orifice 4, dont la fermeture rapide permet un réglage du débit et par conséquent de la pression du fluide sous pression. La fermeture rapide 6 permet également de fermer cette arrivée de fluide sous pression.

Dans le cas d'utilisation d'air comprimé, un tuyau arrivant sur la fermeture rapide 6 peut être branché sur le circuit d'air comprimé de la salle d'opération. Si un tel circuit fait défaut, il est possible d'utiliser une pompe ou un compresseur autonome.

La pression du fluide dans la seconde chambre étanche gonfle cette dernière et exerce une pression, par le biais des parois intermédiaires, sur la première chambre étanche, et par conséquent sur le liquide aseptique 17 que celle-ci contient. Le chirurgien ou son assistant(e) peut régler la pression au moyen de la fermeture rapide 6. Dans le cas d'un dispositif amenant l'air comprimé par une pompe ou un compresseur, la pression est également réglable à partir de ces éléments.

De préférence, l'ensemble formé par les deux chambres étanches est constitué de quatre parois en matière plastique transparente qui sont réunies par leur pourtour.

La sécurité est un élément essentiel dans tous les dispositifs à usage chirurgical. Dans le présent dispositif, il est important d'éviter tout risque d'infiltration du fluide sous pression, et particulièrement d'air comprimé, dans le liquide aseptique 17, même si un tel risque est minime. C'est à cette fin que la forme d'exécution préférée décrite ici présente deux parois intermédiaires 14 au lieu d'une seule. Dans la forme d'exécution préférée, une extrémité d'un tuyau semi-souple 15 est placée entre les deux parois 14. Son autre extrémité, qui est à l'air libre, présente un élément d'alarme acoustique 18. Cet élément d'alarme acoustique est de préférence un sifflet. Si de l'air s'échappe de la seconde chambre étanche dans l'interstice entre les parois 14, cet air s'écoulera de l'intérieur vers l'extérieur par le tube 15 et parviendra au sifflet, donnant ainsi l'alarme.

Le tuyau 15 sort de l'interstice entre les parois 14 par un point 16 situé sur le pourtour de jonction des deux parois intermédiaires 14. De la sorte, le tuyau 15 ne traverse pas les chambres étanches 12 et 13.

D'autres dispositifs d'alarme pourraient être utilisés, notamment des systèmes qui, par l'intermédiaire d'un senseur, transformeraient la différence de pression en une variation de courant ou de potentiel électrique, qui déclencherait à son tour une alarme sonore ou visuelle. De tels systèmes sont cependant moins simples que celui décrit ci-dessus.

Dans une forme préférée de l'invention, les surfaces des parois 14 qui sont vis-à-vis l'une de l'autre présentent une granulosité, de préférence irrégulière, de manière à éviter qu'elles adhèrent l'une à l'autre. En effet, il n'est pas exclu, dans le cas d'une fuite d'air de la chambre 13 dans l'interstice entre les parois 14, qu'il se forme une cloque entre ces doubles parois, sans que la pression soit suffisante pour que l'air s'échappe par le tuyau 15. En pareil cas, il pourrait éventuellement arriver que la pression soit néanmoins suffisante pour que des bulles d'air s'introduisent dans le liquide 17. La granulosité des parois garantit une circulation de l'air dans l'ensemble de l'espace intersticiel, et par conséquent une répartition uniforme de la pression. Toute surpression engendrerait alors un écoulement à travers le tube 15, et par conséquent le fonctionnement de l'alarme acoustique 17.

Comme dans les dispositifs classiques, le réservoir constitué des deux chambres étanches est suspendu verticalement, les parois étant placées sensiblement verticalement. Il est toutefois concevable de les placer différemment, par exemple horizontalement, le premier sac étanche étant placé au-dessous du second, de façon que le liquide aseptique puisse s'écouler aisément.

## Revendications

1. Dispositif de nettoyage des plaies en chirurgie, comprenant au moins une première chambre étanche (12) apte à contenir un liquide aseptique (17) de nettoyage, un tuyau (5) destiné à conduire le liquide à au moins une buse de giclage, la première et la seconde chambre étant séparée l'une de l'autre par au moins une paroi souple (14), le dispositif comprenant en outre des moyens d'introduire un fluide sous pression dans la seconde chambre et d'exercer ainsi une pression sur la première chambre par l'intermédiaire de la ou des parois souples et par conséquent sur le liquide aseptique (17) contenu dans la première chambre étanche, caractérisé en ce que les parois des deux chambres sont entièrement souples.

2. Dispositif selon la revendication 1, caractérisé en ce que les deux chambres étanches et la ou les parois qui les séparent sont constituées de parois étanches réunies par leur pourtour.

3. Dispositif selon la revendication 2, caractérisé en ce que les parois des chambres étanches sont planes lorsque les chambres sont vides.

4. Dispositif selon l'une des revendications 1, 2 ou 3, caractérisé en ce que l'orifice (4) est conformé de façon à pouvoir être branché sur un circuit d'air comprimé.

5. Dispositif selon l'une des revendications 1 à 4, caractérisé en ce que les première et seconde chambres étanches (12, 13) sont séparées par au moins deux parois souples (14) et en ce que le dispositif comprend en outre un élément d'alarme (18) apte à fonctionner en cas d'intrusion de fluide entre lesdites parois souples (14) de séparation.

6. Dispositif selon la revendication 5, caractérisé en ce que l'élément d'alarme (18) est acoustique.

7. Dispositif selon les revendications 4, 5 et 6, caractérisé en ce que l'élément d'alarme est un sifflet.

8. Dispositif selon l'une des revendications 5, 6 ou 7, caractérisé en ce qu'au moins une des faces des parois souples (14) de séparation qui sont en vis-à-vis a une surface granuleuse.
